# EUROPEAN PATENT APPLICATION

(11) **EP 1 092 778 A1**
(43) Date of publication of application: **18.04.2001**
(21) Application number: 99870208.8
(22) Date of filing: 13.10.1999
(51) Int. Cl.: C12N 15/82

(54) **Agrobacterium-based transformation method for Beta vulgaris**

(71) Applicant: Ses Europe N.V./S.A., 3300 Tienen (BE)
(72) Inventor: The designation of the inventor has not yet been filed
(74) Representative: Van Malderen, Michel

(57) **Abstract**

The present invention is related to a method of genetic transformation of a plant or plant cells, comprising the step(s) of introducing an hereditary material into callus derived from the guard cells of said plant or said plant cells by an *Agrobacterium* vector and possibly regenerating whole plant from said transformed plant cells.

## Description

### Field of the invention

The present invention is related to a new transformation method for plants, especially *Beta vulgaris,* which includes sugar beet, fodder beet, table beet and Swiss chard. Said method comprises a method for the genetic transformation of plant cells, the regeneration of whole plants from such plant cells and the plants so obtained, including plant tissue such as fruit, stem, root, tuber or seeds of said plants.

### Background of the invention and state of the art

The quest for an efficient method for the genetic modification of *Beta vulgaris* has been going on for more than 15 years. Sugar beet could benefit greatly from such a method in that many crop limitations could effectively be overcome using a biotechnological approach. For example, aspects such as bolting, resistance and quality could be tackled using genes not available in the restricted genetic pool of *Beta vulgaris* and its sexually compatible relatives. In addition, with its phenomenal capacity for sucrose production (approximately 75% root dry weight) there is increasing interest in exploiting sugar beet as an environmentally-friendly and economically viable bioproduction facility for alternative carbohydrates (Sevenier).

For many years, and despite intensive efforts, no system for the efficient genetic modification of *Beta vulgaris* was available. The publication **[1]** describes a protoplast / Poly Ethylene Glycol (PEG) based transformation method that enables the production of sufficient numbers of transgenic plants in a single experiment. This method, being based upon a specific cell type (the stomatal guard cell), could be directly optimised for every step in the transformation protocol so that a method could be developed which gives exclusively transgenic regenerants and no chimeric plants or true escapes. However, when these plants are to be used for breeding purposes a subsequent molecular screening is necessary to eliminate those plants that have more than the desired number of transgenes as a result of the use of a direct DNA transfer system. Such an elimination can entail the loss of less than 50% of the transformed regenerants. Nevertheless, such a method can still be effectively exploited commercially and, regarding novel applications for sugar beet, it has already made possible the production of the first fructan-accumulating transgenic beet, the Fructan Beet® **[2]**.

An *Agrobacterium*-based transformation system for sugar beet could have a number of additional advantages. The most important of these is the potential to avoid the production of highly multi-copy transgenics as is frequently found when PEG is used. This would enhance the frequency of potentially useful plants containing ≤4 copies of the transgene. This is of particular importance in sugar beet where it has been found by two independent research groups that there is a clear negative correlation between copy number and transgene expression **[3, 4]**. Previously, attempts to transform sugar beet using *Agrobacterium* vector have generally had little or no success. Recently, a first method has been described in detail and is based upon the use of cotyledon explants together with a selection system based on glyphosate **[4]** or mannose **[5]**. Said method is clearly functional although a distinct genotype-dependence was detected and on average, some 14000 individual cotyledons are needed to obtain 100 putative transgenics **[4]**.

There has been considerable speculation as to why the *Beta vulgaris* transformation capacity could generally not be combined with the subsequent regeneration of transgenic sugar beet plants. A possible physical barrier between the cells which can be induced to regenerate (with)in sugar beet explants and the inoculating agrobacteria, or the possibility that cells with the combined competence for both regeneration and transformation may be difficult to obtain, have been widely discussed.

The document W095/10178 describes a method of producing a plant, especially *Beta vulgaris,* comprising culturing a stomatal cell in a regeneration method, this stomatal cell being isolated from leaf epidermis of said plant tissue. In said method, the stomatal cells are preferably converted to protoplasts prior to regeneration.

### Aims of the invention

The main aim of the present invention is to provide a new transformation method for plants, especially *Beta vulgaris,* which does not present the drawbacks of the methods of the state of the art.

A further aim of the present invention is to provide such a method that allows the genetic transformation of plant cells, the regeneration of the whole plants from such cells and the plants so obtained, which is easier to obtain and less labour-intensive than the methods of the state of the art, and which may reduce or eliminate the risks of contamination of the starting materials or the plants so obtained.

Another aim of the present invention is to provide such a method that allows a higher regeneration efficiency in combination with a shorter culture regime.

A last aim of the present invention is to provide such a method that allows a more controlled gene integration (reduced copy number of the transgene) than the methods of the state of the art.

### Summary of the invention

The present invention is related to a method of genetic transformation of a plant or plant cells, comprising the steps of introducing a hereditary material (a nucleotidic sequence) into callus derived from guard cells of said plant or said plant cells by an *Agrobacterium* vector and possibly regenerating a whole plant from said transformed plant cells.

The method for the regeneration of a plant, preferably a sugar beet, from transgenic callus derived from guard cell protoplasts according to the invention is based upon the use of *Agrobacterium* (*Agrobacterium* plasmid vector, preferably the one described in the following description) as the agent for the transfer of hereditary material (DNA sequence) instead of PEG. Such a method has a number of clear advantages not only in giving more controlled gene integration but also in e.g. providing an alternative genetic modification system for recalcitrant lines. The risk of transgenic chimeras, as can arise from an explant-based system, can be avoided by exploiting the somatic embryogenic potential of guard cell-derived callus and also the labour input needed to obtain appropriate numbers of transgenic plants for each gene construct could be significantly reduced.

Preferably, the method according to the invention is related to a method of transformation of *Beta vulgaris,* which includes sugar beet, fodder beet, table beet and Swiss chard.

In the method according to the invention, the transfection is increased in the presence of methionine and/or acetosyringone both being at a concentration of about 100 µM in the culture medium.

In the method according to the invention, the incubation temperature during cocultivation of guard cells is preferably in the range of about 21 to about 28 °C.

The present invention concerns also the plant or plant cell obtained by the method according to the invention, preferably a *Beta vulgaris* plant or *Beta vulgaris* plant cell, and the plant tissue such as fruit, stem, root, tuber or seeds of said plant.

Advantageously, said plant or said plant cell comprises also a pesticide, herbicide or fongicide resistance.

Preferably, said resistance is selected from the group consisting of nematode or viral resistance (preferably the BNYV resistance obtained for instance by one of the methods described in the document W098/07875 or in the International Patent Application PCT/BE99/00089 incorporated herein by reference), glyphosate resistance, glufosinate resistance and/or acetochloride resistance.

The present invention will be described in the following non-limiting examples.

### Detailed description of the invention

### Material and methods

### Guard cell protoplast isolation

Two distinctly different sugar beet breeding lines (populations) were used: Bv-NF derived from a breeding programme at CPRO-DLO, The Netherlands and SES-345 which was derived from a breeding programme at Advanta, Belgium. Guard cell protoplasts were generally isolated as described **[7]** with slight modifications. Briefly, 1.5 g deribbed leaf material was harvested from 4-week-old shoot cultures and was blended in a Waring blender together with 50 ml sterile tap water (4 °C) at maximum speed for 60 sec. The clean epidermal fragments were collected on a 297 µm filter and washed with 200 ml sterile tap water before transferring to 10 ml preincubation medium **[6]** for 1 hour. Cell wall digestion took place overnight in CPW9M medium supplemented with 0.5% Cellulase RS and 3% Cellulase R10. Protoplast purification was exactly as described previously **[7]**. Using this protocol, 2 x 10⁶ protoplasts per g deribbed leaf are obtained of which 80-95% are viable guard cell protoplasts.

### Guard cell protoplast culture

Before culture, aliquots of 62500 protoplasts were embedded in 1 ml 1% calcium alginate in a thin layer (<1 mm thick, 5-6 cm diameter) as described previously **[7]**. These alginate discs were cultured in liquid K8P medium **[8]** modified as described **[9].** Plating efficiencies of 20-30% can be expected in these cultures with cell division beginning on approximately day 7 and by day 18 colonies are visible to the naked eye. At this time the alginate disc was cut into strips and these were transferred to PG1B medium **[7]** solidified with 0.9% agar (Daichin, Tokyo, Japan). The transformation procedure began 7 days later by which time the colonies were already emerging from the upper surface of the alginate and had formed a layer of almost touching colonies over the entire area of the alginate strips.

### Transformation

A number of *Agrobacterium* strains were used each containing the same binary vector. For negative controls the same strains but lacking the binary vector were used. The strains employed were AGL 0, LBA 4404 and a methionine auxotrophic mutant of LBA 4404 denoted HAT 5000. The binary vector used was pPG 6 which was derived from pBIN 19 and contained, within the T DNA region, a *uidA*-intron gene for histochemical GUS staining and also the *bar* gene which confers resistance to glufosinate-type herbicides. Each gene was driven by a CaMV 35S promoter and had a CaMV terminator. Both LBA 4404 strains also contained the pTOK 47 plasmid for enhanced virulence. All bacteria were grown overnight in LB medium supplemented with 25 mg/l streptomycin and 100 µM acetosyringone. Additional supplements of 2 mg/l tetracyclin (for pTOK47) and 100 mg/l kanamycin (for pPG 6) were used where appropriate. Before transformation the bacterial cultures were centrifuged at 3000 rpm for 15 min and the pellet was resuspended in sufficient PGo medium **[10]** supplemented with 100 µM acetosyringone to give the desired inoculum density in the range OD₅₅₀ 0.1-1.0.

For inoculation, the bacterial suspension was poured into a Petri dish in which the alginate strips containing the emerging guard cell derived callusses were submerged, callus side downwards. After 10 min, the strips were removed individually and excess suspension was allowed to drip off before being placed on cocultivation medium with the callus side upwards. The cocultivation medium comprised PG1B medium **[3]** supplemented with 100 µM acetosyringone, 100 µM methionine and 0.9% Seaplaque Agarose, pH 5.8. Cultures were incubated in the dark at 28 °C for 2 d, after which the strips were transferred to selection medium comprising PG1B medium supplemented with 125 mg/l cefotaxime, 1 mg/l bialaphos and 0.8% agarose, pH 5.8. Two days later representative pieces of alginate were removed for histochemical GUS staining and after a further 2 d individual callusses (<0.5 mm) were transferred to 9 cm Petri dishes containing 20 ml fresh selection medium in grids of 10 x 10. Two weeks later, putative transgenic callusses had reached a size of 3-4 mm and were transferred to PNB medium, 20 calli /20 ml medium/9 cm Petri dish. Non-transformed callusses retained their original size on this medium. At the same time, small fractions of the transferred callusses were used for histochemical GUS analyses. PNB medium consisted of PGo medium supplemented with 0.2 mg/l NAA, 1 mg/l BAP, 2 mg/l glycine, 125 mg/l cefotaxime and 0.3% Phytagel (Sigma, St Louis, USA). All calli on PNB medium were cultured in the light at 25 °C and were transferred to fresh dishes every 14 d. Embryos usually formed within the first 6 weeks in the light (2500 lux, Philips Warmwhite fluorescent) and these were grown further as described previously **[3]**.

The *uidA* gene was used to assist in monitoring the effectiveness of the genetic modification treatments tested to enable the optimisation of an appropriate protocol. Before the detailed analysis, initial experiments were performed to confirm that *Agrobacterium* was indeed capable of infecting guard cell derived callus of sugar beet and that the cultures remained viable. Results indicated that these cells readily showed a considerable degree of blue staining with the histochemical GUS test, even before any optimisation, and that the inoculated cultures continued to grow (results not presented). Consequently, experiments were performed using young callusses approximately 3 weeks old to determine the optimum conditions for obtaining maximum levels of transfection of the exposed cells.

### Transient uidA expression

The treatment giving maximum levels of transient GUS activity (2 days after cocultivation) was to be chosen as the ideal starting material for obtaining stably transformed calluses for regeneration. For the three bacteria strains, different inoculum densities and times were tested, initially using the sugar beet line Bv-NF and subsequently SES 345. Using HAT 5000, the highest inoculum density tested (OD 1.0) repeatedly gave the highest degree of blue staining. A higher density resulted in bacterial overgrowth / toxicity problems. This inoculum density was chosen to determine the influence of acetosyringone (to stimulate agroinfection) and methionine (to stimulate growth of the bacterial auxotroph).

### Example 1

The influence of different concentrations of acetosyringone and methionine in the cocultivation medium on the success of Agrobacterium transfection (strain HAT 5000, inoculum density OD₅₅₀,1.0) of guard cell derived callus of sugar beet (line Bv-NF) was determined. Callusses were cocultivated in the presence of agrobacteria for 2 days and two days later transient expression (TE) of the *uid A* gene was visually estimated in terms of the fraction of the total callus surface area which stained blue (range: (+) individual blue cells / small clusters; + blue areas < 25% total s.a.; ++ blue areas 25 - 50% total s.a.; +++ blue areas 50 - 80% total s.a.). Stable transgene integration (S) was determined after the transfer of 200 individual callusses to selection medium. The percentage of actively growing callusses was determined after 2 - 3 weeks. Results (Table 1) indicated that while in all cases transient *uidA* expression was evident there was an optimum of 100 µM for each supplement.

**Table 1**

| **Methionine (µM)** | **50** | | **100** | | **200** | |
|---|---|---|---|---|---|---|
| **Acetosyringone (µM)** | Transformation | | Transformation | | Transformation | |
| | TE | Stable | TE | Stable | TE | Stable |
| **50** | +++ | 54% | (+) | 80% | (+) | 82% |
| **100** | (+) | 83% | +++ | 90% | + | 66% |
| **200** | (+) | 72% | ++ | n.d. | ++ | 89% |
| n.d.: not determined | | | | | | |

### Example 2

The influence of inoculum density and time of exposure to the Agrobacterium suspension on transient uidA expression in guard cell derived callus cultures of sugar beet line Bv-NF was determined. After a given exposure time to a bacterial suspension the callus cultures were placed on cocultivation medium for 2 days. The cultures were then transferred to selection medium and two days later histochemical GUS analyses were performed. Results are given in terms of the estimated surface area of the callusses that stained blue. At least 3 cm² alginate culture (approximately 3000 individual callusses) were used for each histochemical GUS test.

In this treatment almost the entire exposed surface of the callusses stained positive for GUS. For AGL 0, the highest transfection frequency was found when an inoculum density of 0.5 was used (Table 2).

**Table 2**

| **Agro. Strain** | **Inoculum Density** | **Exposure 10 min** | **Exposure 60 min** | **Exposure 180 min** |
|---|---|---|---|---|
| **HAT 5000** | 1.0 | 60% | 50% | 50% |
| **AGL 0** | 0.1 | 40% | 50% | 50% |
| **AGL 0** | 0.5 | 70% | 70% | 80% |
| **AGL 0** | 1.0 | 20% | 20% | 20% |

Again a higher inoculum density quickly leads to symptoms of toxicity. Agroinfection was very extensive and the period of incubation of the callusses in the bacterial suspension needed only to be short in order to obtain a high level of transient *uidA* expression (Table 2). Equivalent results for the SES 345 line were obtained (Table 3).

### Example 3

The success of transfection of guard cell derived callus of the sugar beet line SES 345 using three different Agrobacterium strains and three different inoculum densities is presented (Tables 3 and 7). Results are presented as a percentage of the total callus surface area stained blue after the histochemical GUS test. At least 3 cm² alginate culture (approximately 3000 individual callusses) was used for each histochemical GUS test.

**Table 3**

| **Agrobacterium strain** | **Inoculum density 0.1** | **Inoculum density 0.5** | **Inoculum density 1.0** |
|---|---|---|---|
| **LBA 4404** | 10% | 40% | 50% |
| **HAT 5000** | n.d. | 50% | 75% |
| **AGL 0** | 20% | 60% | 50% |
| n.d.: not determined | | | |

### Example 4

Effect of the incubation temperature during cocultivation of guard cell derived callus of sugar beet line Bv-NF with AGL 0 and LBA 4404 is presented in the Table 4. Transient GUS activity was determined after 2 days cocultivation and 2 days culture on selection medium. Results are presented in terms of the visually estimated percentage of the surface area of the callusses that stained blue. At least 3 cm² alginate culture (approximately 3000 individual callusses) were used for each histochemical GUS test.

Temperatures in the range 21-28 °C appeared not to influence the success of agroinfection and 28 °C was retained as standard (Table 4).

**Table 4**

| **Temperature** | **Expt1** | | **Expt2** | |
|---|---|---|---|---|
| | **AGL 0** | **LBA 4404** | **AGL 0** | **LBA 4404** |
| **21 °C** | 90% | 90% | 90% | 90% |
| **25 °C** | 90% | 90% | 90% | 90% |
| **28 °C** | 90% | 90% | 90% | 90% |

### Example 5

### Stable transformation

After cocultivation and a brief period on selection medium individual calli were picked off using fine forceps for further culture. Failure to do this resulted in bacterial overgrowth even on medium containing up to 250 mg/l cefotaxime. A bialaphos concentration 4 x that previously used for protoplast cultures was found to be optimal for these callus cultures. Transferring the tiniest amounts of callus possible (<0.5 mm) was necessary in order to ensure maximum effect of the selection agent. Taking larger pieces resulted in 100% of the callusses growing further and all being GUS negative. Callusses taken from the treatment giving optimal transient expression were later also observed to have the highest frequencies of stable *uidA* expression (Table 1). However, all treatments gave acceptable frequencies of stable *uidA* expression and the best resulted in 90% of the transferred callusses that grew on selection medium being GUS positive. The frequency of callusses continuing to grow on selection medium was usually around 30% for both sugar beet lines (Table 5) but could be as high as 95%.

A comparison of the transformation success in terms of both transient expression (TE) and putative stable transgene integration (herbicide resistant callus) following a callus-based transformation system using two sugar beet genotypes and three bacterial strains each containing the same pPG 6 binary plasmid. The results are the means of 4 or 5 experiments.

**Table 5**

| **Sugar beet line** | **Agrobacterium strain** | **No. Expts** | **TE** | **Calli /Expt** . | **Herbicide resistant** | **%Res. Calli GUS+** |
|---|---|---|---|---|---|---|
| SES 345 | LBA 4404 | 4 | 46% | 340 | 28% | 90% |
| | HAT 5000 | 4 | 45% | 300 | 22% | 61% |
| | AGL 0 | 4 | 30% | 325 | 27% | 86% |
| Bv-NF | LBA 4404 | 5 | 74% | 680 | 29% | 81% |
| | HAT 5000 | 5 | 70% | 460 | 16% | 61% |
| | AGL 0 | 5 | 50% | 460 | 29% | 78% |

### Example 6

### Regeneration of (putative) transgenic plants

Regeneration of non-transformed sugar beet (Bv-NF) guard cell derived callus following the standard protocol **[3]** [Control]; modified to fit the subculture regime necessary for Agrobacterium transformation [Modified]; and the modified protocol but with the inclusion of acetosyringone and methionine at the appropriate culture stage. Results are presented as the percentage of individual callusses cultured (200 callusses per treatment) giving rise to an established plantlet.

**Table 6**

| **Treatment** | **Experiment 1** | **Experiment 2** | **Experiment 3** | **Mean** |
|---|---|---|---|---|
| **Control** | 2% | 14% | 7% | 7.5% |
| **Modified** | 3% | 11% | 10% | 7.8% |
| **+ Aceto/ +methionine** | 3% | 11% | 7% | 6.8% |

The use of 125 mg/l cefotaxime in the culture medium routinely resulted in slightly enhanced (up to 2x) regeneration frequencies although this could not be shown to be statistically significant. Attempts to improve the frequency of regeneration by modifying the phytohormone supplement of the medium revealed that while NAA concentration showed no clear link with regeneration the BAP concentration showed in general a positive correlation with the percentage of calli regenerating a plant. Further enhancement of the BAP concentration could further improve the regeneration response in this system. Plantlets arose via somatic embryogenesis and most could be successfully cultured further. Transfer of the plants to soil was routine and was generally 100% successful.

### Example 7

### Analysis of regenerated plants

A series of plants regenerated from transformation experiments using the three different bacterial strains was subjected to Southern analysis. Both GUS positive and GUS negative plants were taken. DNA was digested using Eco RI that has just two restriction sites either side of the complete pat gene cassette. Consequently hybridisation with the pat coding sequence should yield information on the precision of Ti integration and hybridisation with the GUS coding sequence should yield information on likely numbers of transgene integration sites.

The choice of starting material is paramount and the relative uniformity of the guard cell callus system has lead to a protocol that results in the successful transformation of the exposed cells covering almost the entire callus surface. Those cells which do not come into contact with the bacteria, either due to their still being embedded in the alginate carrier or through being within the callus clumps, fail to be transformed. This is also the case with explant systems. However, these cells can be eliminated later by using a modified selection pressure and by keeping the amount of material initially subcultured to a minimum. In this way the occurrence of non-transformed escape plants can be kept at an acceptably low level. The bialaphos-based selection strategy has again been remarkably effective for sugar beet as was previously also found with the protoplast based transformation system **[3]**.

A potential disadvantage of any explant based system is the possibility of obtaining chimeric transgenics through the organogenic formation of regenerated plants from multicelled structures. In the guard cell callus system employed here, regenerants arise strictly via somatic embryogenesis and are therefore, presumably of single cell origin **[3]**. Consequently, an appropriate selection pressure should ensure that only transformed cells give rise to fully transformed plants and to date no indication of chimeric plants arises in this system.

Clearly all three Agrobacterium strains were effective in sugar beet guard cell callus inoculation. Furthermore, both sugar beet lines used, which have notably divergent genetic backgrounds, appeared similarly susceptible to the transformation protocol developed. Preliminary results from experiments using other lines back up the proposal that the use of guard cell derived callusses as a starting material is widely applicable. The relative ease of Agrobacterium infection of this sugar beet tissue has also meant that many of the different treatments tested gave similar results.

GUS activity appears to be a valuable means to estimate the effectiveness of different transformation treatments.

With the method according to the invention, the ability to produce more callusses in a single experiment than is practically usable combined with a selection regime which gives an acceptably low level of escapes, means that the single remaining bottle neck is the relatively low efficiency of plant regeneration. While sufficient plants can be produced for further use, it is clear that improving the regeneration efficiency could significantly increase the productivity of the system and reduce the input required. However, in contrast to a direct DNA transfer system, almost all the plants obtained via the Agrobacterium route are likely to be suitable for further use. Using the PEG-protoplast system some 30% of the regenerants contained large copy numbers of the transgene **[1]**. Such plants are totally absent from the Agrobacterium-based system. The occurrence of rearrangements / partial integration also seems to be significantly reduced.

In comparison with the state of the art, the method according to the invention, which is based on the use of somatic embryogenic callus, has a number of additional advantages to those already described above. It is easier and less labour intensive to obtain the starting material and the risk of contamination is eliminated. Furthermore, the higher regeneration efficiency in combination with a shorter subculture regime (callusses will have regenerated and can be discarded after 6 weeks / 3 subcultures on solid medium) entails that the total input required to obtain an appropriate number of transgenic plants will be considerably less **[5]**. The transfer of large numbers of very small callusses at the start of the procedure is clearly tedious. It is possible to automate this step which will make it possible to perform multiple callus transfers onto selection medium simultaneously.

### Example 8

Regenerated plants obtained in transformation experiments performed using two sugar beet lines and three Agrobacterium strains is presented in Table 7. Only plantlets that could successfully be cultured further were scored. The histochemical GUS test was performed when the plantlets were 1-2 months old. Results are summaries of 4-5 experiments per treatment.

**Table 7**

| **Sugar beet line** | **Agro. strain** | **Resistant callusses cultured** | **Plants regenerated** | **% regeneration** | **% plantlets GUS+** |
|---|---|---|---|---|---|
| **SES 345** | **LBA 4404** | 3609* | 130 | 3.6% | 61% |
| | **HAT 5000** | 456 | 13 | 2.9% | 62% |
| | **AGL 0** | 500 | 5 | 1.0% | 40% |
| **NF** | **LBA 4404** | 853 | 15 | 1.8% | 73% |
| | **HAT 5000** | 351 | 4 | 1.2% | 75% |
| | **AGL 0** | 486 | 11 | 2.3% | 55% |

### REFERENCES

**[ 1]** Hall et al., *Nat. Biotech.* 14 (1996), p. 1133-1138
**[ 2]** Sevenier et al., *Nat. Biotech.* 16 (1998), p. 843-846
**[ 3]** Hall et al., *Nat. Biotech.* 14 (1996), p. 1133-1138
**[ 4]** Mannerlof et al., *Euphytica* 94 (1997), p. 83-91
**[ 5]** Joersbo et al., *Molecular Breeding* 4 (1998), p. 111-117
**[ 6]** Krens et al., *Theoritical and Applied Genetics 79* (1990), p. 390-396
**[ 7]** Hall et al., *Journal of Experimental Botany* 48 (1997), p. 255-263
**[ 8]** Kao and Michayluk, *Planta* 126 (1976), p. 105-110
**[ 9]** Hall et al., *Plant Cell Reports* 12 (1993), p. 339-342
**[10]** de Greef and Jacobs, *Plant Science Letters* 17 (1979), p. 55-61

## Claims

1. Method of genetic transformation of a plant or plant cells, comprising the step(s) of introducing an hereditary material into callus derived from the guard cells of said plant by an *Agrobacterium* vector and possibly regenerating whole plant from said transformed plant cells.

2. Method according to claim 1, wherein the plant is B*eta vulgaris.*

3. Method according to claim 1 or 2, characterised in that the step of introducing an hereditary material into the guard cell derived callus by an *Agrobacterium* vector is made in the presence of methionine and/or acetosyringone both being at the concentration of about 100 µM in the culture medium.

4. Method according to any of the preceding claims, characterised in that the step of introducing an hereditary material into guard cell derived callus by an *Agrobacterium* vector is made with an incubation temperature comprised in the range of about 21 °C to about 28 °C.

5. Plant or plant cell obtained by the method according to any of the preceding claims being preferably a *Beta vulgaris* plant or *Beta vulgaris* plant cells.

6. Plant or plant cell according to claim 5, characterised in that it further comprises a pesticide, herbicide or fongicide resistance.

7. Plant or plant cell according to claim 6, characterised in that said resistance is selected from the group consisting of nematode or viral resistance, glyphosate resistance, glufosinate resistance and/or acetochloride resistance.

8. Plant tissue selected from the group consisting of fruit, stem, root, tuber or seeds of a plant according to claim 5 to 7.
